# EUROPEAN PATENT SPECIFICATION

(11) **EP 2 654 861 B1**
(45) Date of publication and mention of the grant of the patent: **19.08.2015**
(21) Application number: 11810583.2
(22) Date of filing: 23.12.2011
(51) Int. Cl.: A61M 5/44, A61M 5/168, A61M 5/172

(54) **FLUID CIRCULATION SYSTEM**
FLUIDZIRKULATIONSSYSTEM
SYSTÈME DE CIRCULATION DE FLUIDE

(30) Priority: 23.12.2010 GB 201021898
(43) Date of publication of application: 30.10.2013
(73) Proprietor: Biosurgical S.L., Madrid 28864 (ES)
(72) Inventor: ALBALAT, Alberto Martinez, Madrid 28864 (ES)
(74) Representative: Jacob, Reuben Ellis
(86) International application number: PCT/EP2011/006617
(87) International publication number: WO 2012/084268

(56) References cited:
- WO-A2-2008/048189
- US-A1- 2008 146 996

## Description

The present invention relates to a system and method for the delivery of heated therapeutic fluids.

Chemotherapy can use several kinds of drugs, for instance cytotoxic drugs to destroy cancer cells. Commonly the cytotoxic drugs are injected directly into a patient's bloodstream or are provided in the form of tablets or capsules that breakdown such that the cytotoxic drugs enter the patient's bloodstream indirectly. Such techniques rely on the cytotoxic drugs circulating within the patient's bloodstream to reach the cancer cells.

Chemotherapy can be used as only treatment but also associated or combined with several other treatments, like cytoreductive surgery, radiotherapy and others, as a regular approach to several cancer combined therapies.

The use of heat as a treatment, commonly known as hyperthermia, used in combination with any of the previously mentioned treatments, but especially in combination with certain chemotherapic drugs has shown promising results in several clinical studies.

Bladder cancer may be treated by cytotoxic drugs being delivered directly to a patient' bladder, which is known as intra-vesical chemotherapy. This technique delivers the cytotoxic drugs directly to the cancer cells with minimal absorption of the cytotoxic drugs into the patient's bloodstream. This means that intra-vesical chemotherapy is associated with higher effectiveness and fewer side effects than techniques that rely on the cytotoxic drugs circulating within the patient's bloodstream.

Some other cancers, like ovarian cancer that has spread to the peritoneum and peritoneal mesothelioma may be treated by cytotoxic drugs being put directly into a patient's abdomen during or after surgery, which is known as cytoreductive surgery. As with intra-vesical chemotherapy, this technique delivers the cytotoxic drugs directly to the cancer cells with minimal absorption of the cytotoxic drugs into the patient's bloodstream.

In intra-vesical and intra-peritoneal chemotherapy, the cytotoxic drugs are delivered to a patient's bladder or abdomen in the form of fluids via a catheter(s). In intra-vesical chemotherapy, the catheter(s) are inserted via a patient's urethra, whereas in intra-peritoneal chemotherapy the catheter(s) are inserted via a hole cut in the wall of a patient's abdomen or by laparoscopic techniques. The fluids may be added to a patient's bladder or abdomen using a first catheter (one or multiple) allowed to circulate within the bladder or abdomen and then withdrawn from the bladder or abdomen using a second catheter (one or multiple). The cytotoxic drugs circulated within the bladder or abdomen may be heated to a few degrees above body temperature to make the drugs more effective in killing the cancer cells, this is known as hyperthermia.

Hyperthermia has been shown therapeutic effect on killing tumoral cells, since normally tumoral cells are more sensitive and less resistant to temperature increase compared to normal cells, but also to alter distribution of several drugs (increase absorption). In particular, hyperthermia has been shown to increase drug uptake by neoplasic cells while at the same time inhibiting DNA repair in damaged neoplasic cells.

Clearly, it is important that the heating of any therapeutic fluid for circulation within a patient's body cavity be very tightly controlled. If a therapeutic fluid within a patient's body is circulated at too high a temperature the patient's tissues may be damaged locally. Increase of central body temperature over certain values can also result in serious complications, systemic damages and potentially fatal complications for the patient. Equally if a therapeutic fluid within a patient's body is circulated at too low temperature the therapeutic fluid may not be as effective or, in extreme circumstances, hypothermia and thermal shock can result.

US 2008/0146996 describes a system for infusing a fluid into a patient's body, for example for infusing blood into a patient's circulatory system. The system comprises a heat exchanger for warming or cooling the fluid prior to infusion and a number of sensors for monitoring the temperature and pressure of the fluid before, during and after passage through the heat exchanger.

The present invention provides an improved system for the delivery and/or recirculation of heated therapeutic fluids. The system of the present invention is preferably used for the delivery of heated therapeutic fluids to the bladder, or peritoneum, but can also be used for the delivery of heated therapeutic fluids to other organs, such as the kidneys, colon, or the liver.

According to a first aspect of the invention, there is provided a system for the delivery of heated fluid to a patient's body cavity, said system comprising a heating element and a pump; and a tubing system, said tubing system comprising at least one length of tubing, a heat exchanger and at least one in-line temperature sensor. The arrangement is such that, in use, the fluid is heated by the heating element and pumped through the tubing system so that it is delivered to the patient's body cavity. The tubing system further comprises at least one in-line temperature sensor located outside the patient's body cavity such that, in use, the temperature sensor measures the temperature of the fluid as it enters the patient's body cavity and/or at least one in-line temperature sensor located outside the patients body cavity such that, in use, the temperature sensor measures the temperature of the fluid as it exits the patient's body cavity.

The system may comprise an integrated control unit that controls the heating and/or pumping element.

Preferably, the heating element comprises heating means. More preferably, the heating element comprises both heating and cooling means.

The heating element may comprise means for controlling the temperature of the fluid. Preferably, means for controlling the temperature of the fluid is adapted to adjust the temperature with a variation of at most +/- 1°C.

The tubing system may further comprise at least one pressure sensor.

The system may further comprise a patient cavity sensor located such that, in use, the temperature sensor measures the temperature in the patient cavity.

The system may be adapted to enable the re-circulation of the heated fluid delivered to the patient's body cavity, such that the heated fluid is pumped through a circuit comprising the patient's body cavity, a first length of tubing, the heat exchanger, and a second length of tubing.

The tubing system may comprise at least one, and preferably two, in-line temperature sensors, wherein the at least two in-line temperature sensors are located such that, in use, one measures the temperature of the fluid as it enters the patient's body cavity and one measures the temperature of the fluid as it exits the patient's body cavity, and wherein the heating element is controlled in response to the temperatures measured by the at least two in-line temperature sensors.

To illustrate the invention, there is described a method, not claimed, of treating a patient using heated fluids within a patient's body cavity, said method comprising the steps of heating the fluid using a device comprising a heating element and a pump; and pumping the fluid so that it is delivered to the patient's body cavity by means of a tubing system comprising at least one length of tubing, a heat exchanger and at least one in-line temperature sensor.

An integrated control unit may be used to control the heating and/or pumping element.

Preferably, the heating element both heats and cools the fluid using heating means and cooling means.

The method may further comprise the step of measuring the pressure of the fluid within the tubing system.

The method may comprise the additional steps of:
c) withdrawing the fluid delivered to the patient's body cavity by means of a first length of tubing; and
d) pumping the fluid through the heat exchanger and a second length of tubing so that is re-delivered to the patient's body thereby re-circulating the heated fluid.

Preferably, during step b) the temperature of the fluid being delivered to the patient's body cavity is measured using a first in-line temperature sensor, and during step c) the temperature of the fluid withdrawn from the patient's body cavity is measured using a second in-line temperature sensor, thereby enabling the heating element to be controlled in response to the temperatures measured by the two in-line temperature sensors.

The invention will be further described with reference to the accompanying figures, in which:
figure 1A and 1B are diagrams of a system according to the invention;

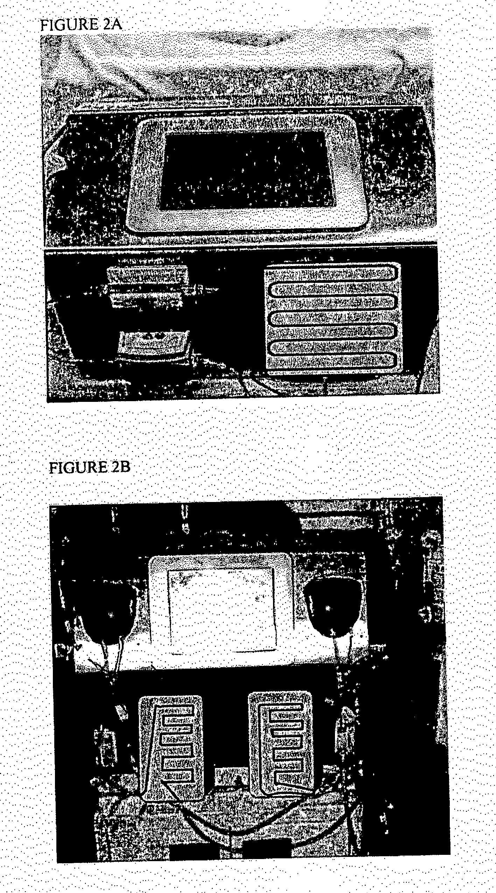

figures 2A and 2B are front view photographs of the electronic units to be used with the system shown in figure 1A and 1B;

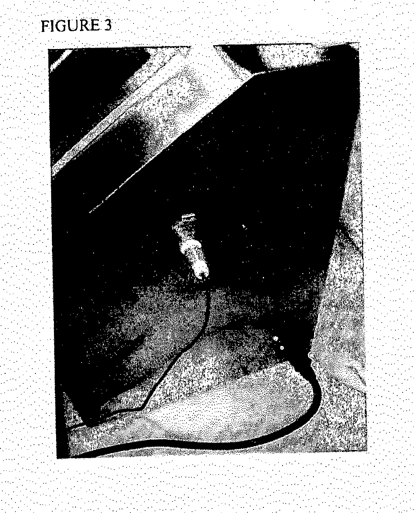

figure 3 is a partial side view photograph of the system shown in figure 2A;
figures 4A and 4B are photographs of a pressure sensor for use with a system according to the invention, shown with and without the tubing system, respectively;
figure 5 is a photograph of a silicone tube in a peristaltic pump for use with a system according to the invention;
figure 6A is a schematic representation of the in-line temperature probe/sensor according to the invention and 6B and 6C are photograph and schematic representation respectively of the end of the temperature sensor connected to a system according to the invention; figures 7A and 7B are schematic representations of a system according to the invention, shown with and without the tubing system, respectively;

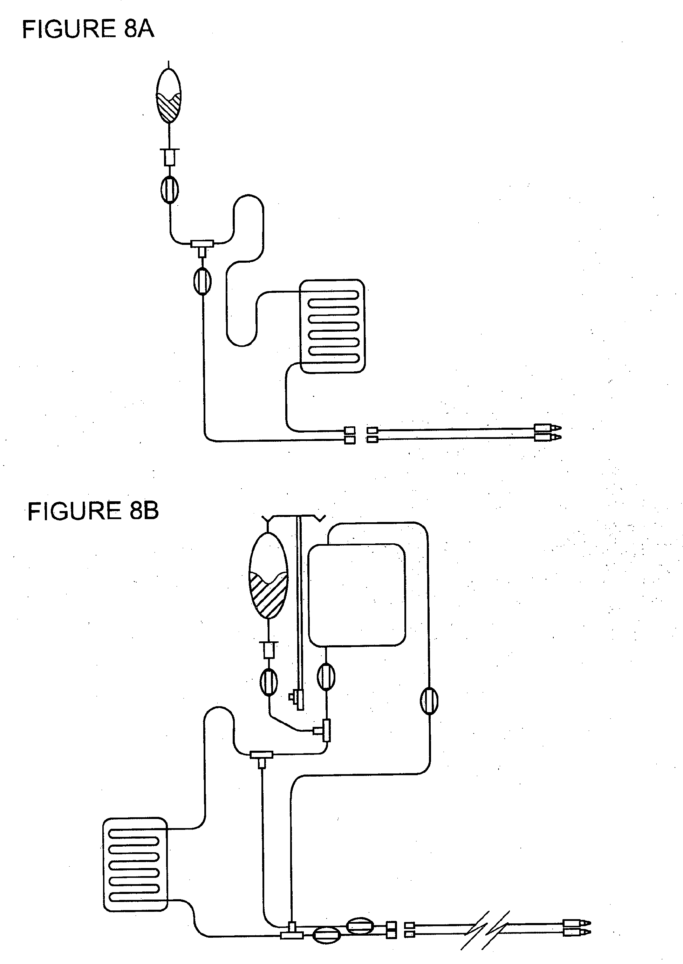

figures 8A & 8B are schematic representations of the tubing system for peritoneum treatments according to the inventions; and
figures 9A & 9B are schematic representations of the tubing system for bladder treatments according to the inventions.

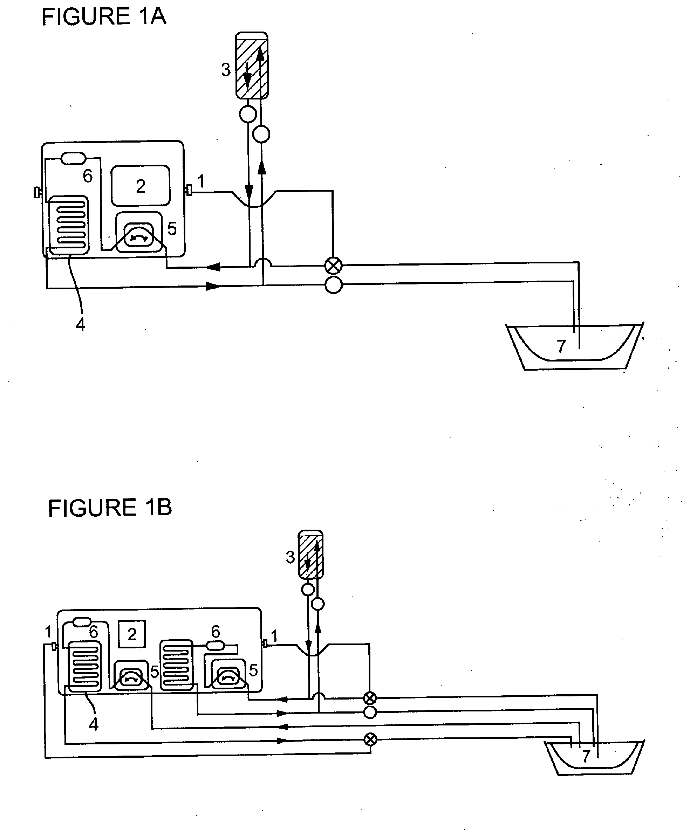

Figure 1A and 1B are diagrams of a system according to the invention. The reference numerals designate the following components: 1. Temperature sensor; 2. Display; 3. Circulating solution/drug; 4. Heat exchange unit; 5. Peristaltic pump; 6. Pressure sensor and 7. Body cavity.

The heating element is used to heat the heat exchanger, which heat exchanger is connected to the at least one length of tubing such that the therapeutic fluid is heated as it passes through the heat exchanger. Suitable materials for making the heat exchanger have to be biocompatibles but also compatibles with the fluid to be circulated, as for example specialized medical grade plastics, and should preferably contain high conductive materials for instance medical grade aluminium.

Preferably, the heating element is located in a warming unit. The tubing system is in fluid communication with a port, which may be attached to a bag or a syringe through which the therapeutic fluid and is connected to the heat exchanger. Suitable materials for the tubing are any biocompatible material, also compatible with the fluid to be circulated, and preferably low heat conductive material to minimize heat lose as for example medical grade silicone.

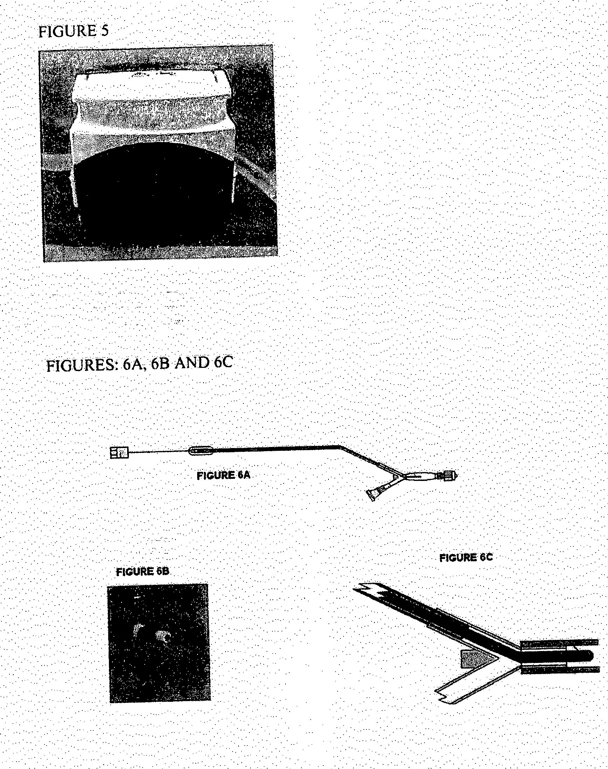

In intra-vesical chemotherapy the pump will typically maintain a recirculation flow of between 1ml/min to 3,000ml/min. This flow may be obtained by means of at least one peristaltic pump (see figure 5) working at the required flow rate.

Commonly, the tubing system will be designed for single use and will be supplied as a sterile consumable. Manufactured in a compatible material for its intended use.

Typically, the system will comprise an integrated control unit that controls the heating element. Preferably the integrated control unit will contain a control panel and a screen, which screen may display real-time temperature readings. The integrated control unit may also enable the recording of data (e.g. to record the length of treatment and the temperature profile of the treatment) and may also enable the programming of the system (e.g. a user may choose a program with a specific length of and temperature profile for treatment of a patient).

Any suitable type of heating elements may be used to heat the therapeutic fluids, however, heating elements that can accurately control temperature are preferred.

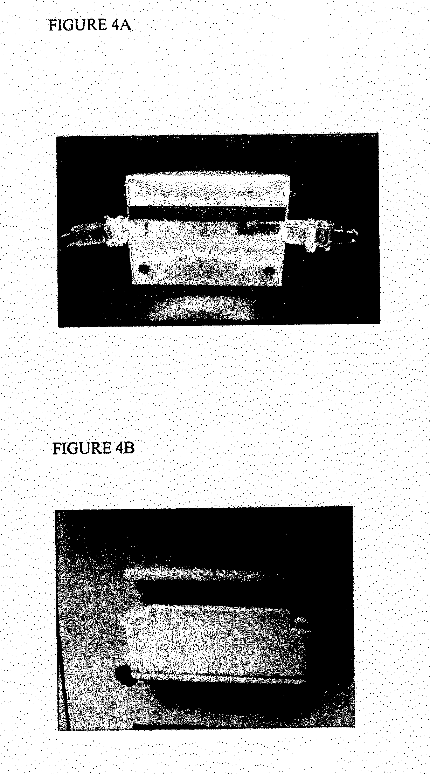

A system according to the present invention may additionally comprise at least one pressure sensor (see figures 4A, 4B) for measuring the pressure of the therapeutic fluid within the tubing system and/or body cavity.

In one embodiment of the present invention, the system is adapted to enable the re-circulation of the heated therapeutic fluid delivered to the patient's body cavity, such that the heated therapeutic fluid is pumped through a circuit comprising of the patient's body cavity, a first length of tubing, the heat exchanger, and a second length of tubing.

In one embodiment of the present invention, the system comprises at least one in-line temperature sensor which, in use, measures the temperature of the therapeutic fluid as it enters the patient's body cavity. For example, the system may comprise only one temperature sensor, which is an in-line temperature sensor which, in use, measures the temperature of the therapeutic fluid as it enters the patient's body cavity.

In another embodiment of the present invention, the system comprises at least one in-line temperature sensor which, in use, measures the temperature of the therapeutic fluid as it exits the patient's body cavity. For example, the system may comprise only one temperature sensor, which is an in-line temperature sensor which, in use, measures the temperature of the therapeutic fluid as it exits the patient's body cavity.

In embodiments in which the system is adapted to enable the re-circulation of the heated therapeutic fluid delivered to the patient's body cavity, the system may comprise at least two in-line temperature sensors. The at least two in-line temperature sensors may be located such that, in use, one measures the temperature of the therapeutic fluid as it enters the patient's body cavity and one measures the temperature of the therapeutic fluid as it exits the patient's body cavity, and wherein the heating element is controlled in response to the temperatures measured by the at least two in-line temperature sensors. The in-line temperature sensor that measures the temperature of the therapeutic fluid as it enters the patient's body cavity can be used to ensure that the heating element does not heat the therapeutic fluid to too high a temperature to prevent tissue damage. The in-line temperature sensor that measures the temperature of the therapeutic fluid as it exits the patient's body cavity can be used to ensure that the heating element heats the therapeutic fluid to a high enough temperature to maximise the effectiveness of the therapeutic fluid.

The system of the present invention may further comprise at least one temperature sensor in the patient cavity to measure the temperature in the patient cavity. For example, the system may comprise at least one cavity temperature sensor and at least one in-line temperature sensor at the entry; at least one cavity temperature sensor and at least one in-line temperature sensor at the exit; or at least one cavity temperature sensor, at least one in-line temperature sensor at the entry and at least one in-line temperature sensor at the exit.

One end of the temperature sensor(s) is connected to the system for example as shown in figures 6A, 6B and 6C.

Additionally, in embodiments in which the system is adapted to enable the re-circulation of the heated therapeutic fluid delivered to the patient's body cavity, the tubing system may comprise two catheters. One of the two catheters may be used to deliver the therapeutic fluid to the patient's body cavity and the second of the two catheters may be used to withdraw the therapeutic fluid from the patient's body cavity. The tubing system may also comprise taps that enable the circulation path of the therapeutic fluid to be altered, e.g. the taps may enable the circulation path to switch from a closed loop outside the patient (to allow the therapeutic fluid to heat up until it is within a desired temperature range) to a closed loop passing through the patient's body cavity (to allow the heated therapeutic fluid to circulate within the patient) and to a closed loop outside the patient with an inlet from within the patient's body cavity (to allow the used therapeutic fluid to be withdrawn from the patient once a treatment is completed).

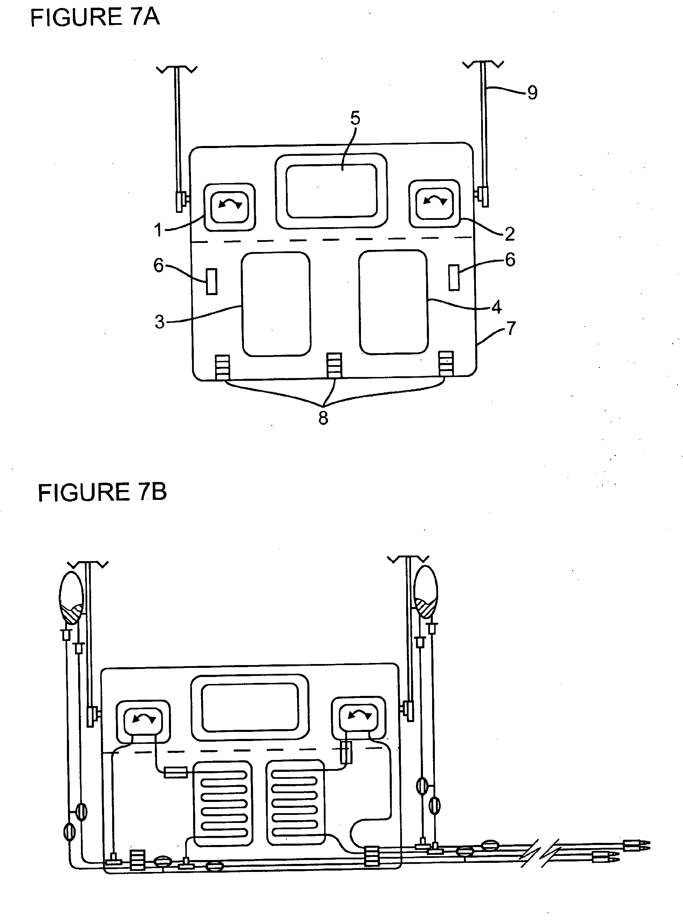

With reference for example to figure 7A, a system according to the present invention may comprise a peristaltic pump processing unit (1), a peristaltic pump (2), a heating system for preheating and recirculation (3) a recirculation heater (4), control panel (5); a pressure sensor (6); a switch button (7); support means for the tubing system (8) and a fluid bag holder (9).

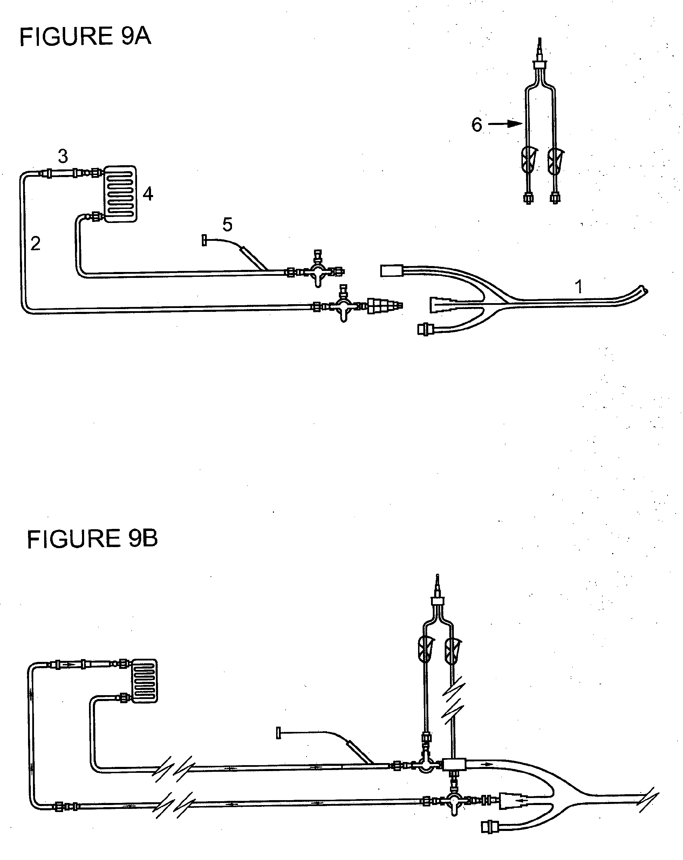

Figures 9A & 9B are schematic representations of the tubing system for bladder treatments according to the inventions. The reference numerals designate the following components: 1. Urologic catheter; 2. Pumping tube segment; 3. Pressure sensor tube segment; 4. Heat exchanger; 5. Temperature sensor; and 6. Irrigation/Drug line.

The method of treating a patient using heated therapeutic fluids within a patient's body cavity comprises the steps of:
a) heating the therapeutic fluid using a device comprising a heating element and a pump; and
b) pumping the therapeutic fluid so that it is delivered to the patients body cavity by means of a tubing system comprising at least one length of tubing, a heat exchanger and at least one in-line temperature sensor.

The method may comprise the additional steps of:
c) withdrawing the therapeutic fluid delivered to the patient's body cavity by means of a first length of tubing; and
d) pumping the therapeutic fluid through the heat exchanger and a second length of tubing so that is re-delivered to the patient's body thereby re-circulating the heated therapeutic fluid.

## Claims

1. A system for the delivery of heated fluid to a patient's body cavity, said system comprising:
a) a heating element 4 and a pump; and
b) a tubing system 2, 3, 4, 6, said tubing system comprising at least one length of tubing, a heat exchanger
the arrangement being such that, in use, the fluid is heated by the heating element and pumped through the tubing system so that it is delivered to the patient's body cavity,
**characterised in that** the tubing system further comprises at least one in-line temperature sensor 5 located outside the patient's body cavity such that, in use, the temperature sensor 5 measures the temperature of the fluid as it enters the patient's body cavity and/or at least one in-line temperature sensor 5 located outside the patients body cavity such that, in use, the temperature sensor 5 measures the temperature of the fluid as it exits the patient's body cavity.

2. A system according to claim 1, wherein the system comprises an integrated control unit that controls the heating element 4 and/or pumping element.

3. A system according to claim 1 or 2, wherein the heating element 4 comprises heating means.

4. A system according to any preceding claim, wherein the heating element 4 comprises heating and cooling means.

5. A system according to any preceding claim, wherein the heating element 4 comprises means for controlling the temperature of the fluid.

6. A system according to claim 5, wherein means for controlling the temperature of the fluid is adapted to adjust the temperature with a variation of at most +/-1°C.

7. A system according to any preceding claim, wherein the tubing system 2, 3, 4, 6 further comprises at least one pressure sensor.

8. A system according to any preceding claim, further comprising a patient cavity sensor located such that, in use, the temperature sensor measures the temperature in the patient cavity.

9. A system according to any preceding claim, wherein the system is adapted to enable the re-circulation of the heated fluid delivered to the patient's body cavity, such that the heated fluid is pumped through a circuit comprising the patient's body cavity, a first length of tubing 2, the heat exchanger 4, and a second length of tubing.

10. A system according to claim 9, wherein the tubing system comprises at least two in-line temperature sensors 5, wherein the at least two in-line temperature sensors are located such that, in use, one measures the temperature of the fluid as it enters the patient's body cavity and one measures the temperature of the fluid as it exits the patient's body cavity, and wherein the heating element 4 is controlled in response to the temperatures measured by the at least two in-line temperature sensors.

## Patentansprüche

1. System zum Zuführen von erhitztem Fluid zu einer Körperhöhle eines Patienten, wobei das genannte System Folgendes umfasst:
a) ein Heizelement 4 und eine Pumpe; und
b) ein Rohrleitungssystem 2, 3, 4, 6, wobei das genannte Rohrleitungssystem wenigstens eine Rohrleitungslänge umfasst, einen Wärmetauscher,
mit einer solchen Anordnung, dass das Fluid beim Gebrauch durch das Heizelement erhitzt und durch das Rohrleitungssystem gepumpt wird, so dass es zur Körperhöhle des Patienten geführt wird,
**dadurch gekennzeichnet, dass** das Rohrleitungssystem ferner wenigstens einen Inline-Temperatursensor 5 umfasst, der sich außerhalb der Körperhöhle des Patienten befindet, so dass beim Gebrauch der Temperatursensor 5 die Temperatur des Fluids misst, während es in die Körperhöhle des Patienten eintritt, und/oder wenigstens einen Inline-Temperatursensor 5, der sich außerhalb der Körperhöhle des Patienten befindet, so dass beim Gebrauch der Temperatursensor 5 die Temperatur des Fluids misst, während es aus der Körperhöhle des Patienten austritt.

2. System nach Anspruch 1, wobei das System eine integrierte Steuereinheit umfasst, die das Heizelement 4 und/oder das Pumpelement steuert.

3. System nach Anspruch 1 oder 2, wobei das Heizelement 4 Heizmittel umfasst.

4. System nach einem vorherigen Anspruch, wobei das Heizelement 4 Heiz- und Kühlmittel umfasst.

5. System nach einem vorherigen Anspruch, wobei das Heizelement 4 Mittel zum Regeln der Temperatur des Fluids umfasst.

6. System nach Anspruch 5, wobei das Mittel zum Regeln der Temperatur des Fluids zum Justieren der Temperatur mit einer Variation von höchstens +/- 1°C ausgelegt ist.

7. System nach einem vorherigen Anspruch, wobei das Rohrleitungssystem 2, 3, 4, 6 ferner wenigstens einen Drucksensor umfasst.

8. System nach einem vorherigen Anspruch, das ferner einen Patientenhöhlensensor umfasst, der so positioniert ist, dass der Temperatursensor beim Gebrauch die Temperatur in der Patientenhöhle misst.

9. System nach einem vorherigen Anspruch, wobei das System so ausgelegt ist, dass das der Körperhöhle des Patienten zugeführte erhitzte Fluid rezirkuliert werden kann, so dass das erhitzte Fluid durch einen Kreislauf gepumpt wird, der die Körperhöhle des Patienten, eine erste Rohrleitungslänge 2, den Wärmetauscher 4 und eine zweite Rohrleitungslänge umfasst.

10. System nach Anspruch 9, wobei das Rohrleitungssystem wenigstens zwei Inline-Temperatursensoren 5 umfasst, wobei die wenigstens zwei Inline-Temperatursensoren so positioniert sind, dass beim Gebrauch einer die Temperatur des Fluids misst, während es in die Körperhöhle des Patienten eintritt, und einer die Temperatur des Fluids misst, während es aus der Körperhöhle des Patienten austritt, und wobei das Heizelement 4 als Reaktion auf die von den wenigstens zwei Inline-Temperatursensoren gemessenen Temperaturen gesteuert wird.

## Revendications

1. Un système de fourniture d'un fluide chauffé à une cavité corporelle d'un patient, ledit système comprenant :
a) un élément de chauffage 4 et une pompe, et
b) un système de tubage 2, 3, 4, 6, ledit système de tubage comprenant au moins une longueur de tubage, un échangeur thermique,
l'agencement étant tel que, en utilisation, le fluide est chauffé par l'élément de chauffage et pompé, au travers du système de tubage, de sorte qu'il soit fourni à la cavité corporelle du patient,
**caractérisé en ce que** le système de tubage comprend en outre au moins un capteur de température en ligne 5 situé à l'extérieur de la cavité corporelle du patient, de sorte que, en utilisation, le capteur de température 5 mesure la température du fluide lorsqu'il entre dans la cavité corporelle du patient et/ou au moins un capteur de température en ligne 5 situé à l'extérieur de la cavité corporelle du patient de sorte que, en utilisation, le capteur de température 5 mesure la température du fluide lorsqu'il sort de la cavité corporelle du patient.

2. Un système selon la Revendication 1, où le système comprend une unité de commande intégrée qui commande l'élément de chauffage 4 et/ou l'élément de pompage.

3. Un système selon la Revendication 1 ou 2, où l'élément de chauffage 4 comprend un moyen de chauffage.

4. Un système selon l'une quelconque des Revendications précédentes, où l'élément de chauffage 4 comprend un moyen de chauffage et de refroidissement.

5. Un système selon l'une quelconque des Revendications précédentes, où l'élément de chauffage 4 comprend un moyen de régulation de la température du fluide.

6. Un système selon la Revendication 5, où le moyen de régulation de la température du fluide est adapté de façon à ajuster la température avec une variation d'au plus ± 1°C.

7. Un système selon l'une quelconque des Revendications précédentes, où le système de tubage 2, 3, 4, 6 , comprend en outre au moins un capteur de pression.

8. Un système selon l'une quelconque des Revendications précédentes, comprenant en outre un capteur de cavité de patient situé de sorte que, en utilisation, le capteur de température mesure la température dans la cavité du patient.

9. Un système selon l'une quelconque des Revendications précédentes, où le système est adapté de façon à permettre la recirculation du fluide chauffé fourni à la cavité corporelle du patient de sorte que le fluide chauffé soit pompé au travers d'un circuit comprenant la cavité corporelle du patient, une première longueur de tubage 2, l'échangeur thermique 4 et une deuxième longueur de tubage.

10. Un système selon la Revendication 9, où le système de tubage comprend au moins deux capteurs de température en ligne 5, où les au moins deux capteurs de température en ligne sont situés de sorte que, en utilisation, l'un mesure la température du fluide lorsqu'il entre dans la cavité corporelle du patient et l'autre mesure la température du fluide lorsqu'il sort de la cavité corporelle du patient, et où l'élément de chauffage 4 est commandé en réponse aux températures mesurées par les aux moins deux capteurs de température en ligne.
